# EUROPEAN PATENT SPECIFICATION

(11) **EP 4 209 484 B1**
(45) Date of publication and mention of the grant of the patent: **15.05.2024**
(21) Application number: 22184081.2
(22) Date of filing: 11.07.2022
(51) Int. Cl.: C07C 253/30, C07C 255/19

(54) **PROCESS FOR PREPARING ELECTRON-DEFICIENT OLEFIN MONOMERS**
VERFAHREN ZUR HERSTELLUNG VON ELEKTRONENARMEN OLEFINMONOMEREN
PROCÉDÉ DE PRÉPARATION DE MONOMÈRES OLÉFINIQUES DÉFICIENTS EN ÉLECTRONS

(43) Date of publication of application: 12.07.2023
(73) Proprietor: Aeardis Ltd., Belfast County Antrim BT2 8DN (GB)
(72) Inventor: MCARDLE, CIARAN, NEWRY, BT35 8DA (GB); YOU, SHULI, SHANGHAI, 200032 (CN)
(74) Representative: Araujo, Daniel

(56) References cited:
- EP-A1- 2 927 209
- WO-A1-2018/114458
- WO-A2-2006/120628
- US-A- 2 330 033

## Description

### Technical Field

The present invention relates to a process for preparing electron deficient olefin monomers comprising nitrile, carboxylic ester or carboxylic acid functional groups, which find application in the field of fast curing adhesives.

### Technical Background

Electron deficient monomers bear two electron withdrawing groups (EWGs) on the same carbon atom of their olefinic bond. These monomers are highly susceptible to polymerisation initiated by electron rich species due to polarisation of their double bond. Cyanoacrylate monomers (CAs) are examples, represented by general formula (I), wherein R is typically an alkyl group. Whereas various CAs are known, only ethyl cyanoacrylate, (ECA) of formula (II) is mass-produced at significant scale due to demand, since it is the major component of commercially important instant adhesives.

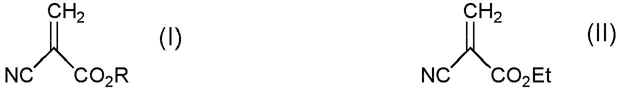

The high reactivity of electron deficient monomers is attractive for application purposes such as rapidly solidifying adhesives, but presents challenges in production, especially at scale. The longstanding traditional production method for CAs exploits a nitrogen-base (N-base/N-based) catalysed Knoevenagel condensation of a cyanoacetate starting material with paraformaldehyde to first form an oligomer that is subsequently thermolyzed back to monomer. Cyanoacetates belong to the class of active methylene compounds, represented by the general formula (III) wherein EWG and Z¹ are electron withdrawing groups that may be the same or different:

EWG-CH₂-Z¹ (III)

For example, ethyl cyanoacetate (NC-CH₂-CO₂Et) is the starting material used to produce ECA.

This class of starting materials is considered 'active' due to the acidity of the central methylene hydrogen atoms that are easily removed by bases. The relative reactivity of active methylene compounds therefore depends on the conditions in which they are deployed as well as the type, strength and combinations of the geminal EWGs they possess, as explained in Sankar et al., Current Chemistry Letts., 2012, 1(3), 123-132.

Cyanoacetates are highly reactive under base catalysed reaction conditions.

The use of various types of amines and ammonium and/or iminium salt organocatalysts in the process of formation of *α,β*-unsaturated compounds by reaction of aldehydes with active methylene compounds in Knoevenagel reactions has been described extensively in general synthetic organic chemistry and, particularly recently in, for example, van Beurden et al., Green Chemistry Letts, and Reviews, 2020, 13(4), 349-364, and in List, B., Angew. Chem. Int. Ed., 2010, 49, 1730-1734. Such N-based organocatalysts activate the active methylene compounds and form carbanion nucleophiles.

A dichotomy arises in the synthesis of electron deficient monomers from active methylene compounds since on the one hand the reactivity of the latter is promoted by bases, but on the other the monomers derived from them are highly unstable when bases are present. In the case of CAs, the traditional production method resolves this problematic situation by first producing oligomers, not monomers, neutralising basic amine catalysts and subsequently thermolyzing or 'cracking' oligomers under acidic conditions to only then release monomer. This two-step process reduces the possibility of free monomer interacting with traces of base. Cracking or thermolysis of abovementioned oligomers is thus an 'indirect' production method since monomer is not obtained from the initial reaction milieu. Similar cracking approaches have been applied to the synthesis of various other electron deficient olefins, for example vinyl diketone and cyanovinyl ketone monomers as described in US 9,828,324 and GB 1,168,000 respectively. However due to commercial importance, most attention using this method has been directed at CA processing as outlined, for example, in US 2,721,858, US 2,756,251, US 2,763, 677, US 4,364,876, US 5,455,369, US 5,624,699, US 6,245,933, and US 9,828,324. Said patents disclose that N-based catalysts specifically promote oligomerisation of starting materials in the synthesis of CAs and vinyl diketones. Said oligomerisation process is explained further in Chorbadjiev et al., Eur. Polym. J., 1991, 27(4/5), 439-443.

Whereas cracking resolves the issue of exposing free monomer to base, the harshness of thermolysis conditions creates disadvantages. This approach is impractical for higher boiling point CA esters and is not applicable at all to the synthesis of monomers with thermally sensitive moieties including monomers with multiple- or mixed-polymerisable functionalities whose oligomers crosslink. Furthermore, the traditional production method for CAs generates a significant quantity of intractable polymer, or so-called crack-residue, that limits monomer yield, is expensive to dispose of and is environmentally unfriendly.

To address some of these shortcomings, efforts have focused on ways to produce electron deficient monomers by 'direct' synthesis and to discover milder conditions to access a broader range of this important class of monomers. Such approaches generally exploit methylenation reactions that involve the formation of a double bonded methylene group (CH₂=) directly on an active methylene compound substrate following transfer of a saturated methylene group (-CH₂-) from a methylene transfer agent. Unlike the traditional indirect approach, direct methods face distinct challenges associated with maintaining stability of the free reactive monomer in the reaction milieux as well as in all subsequent process steps.

In the case of CAs, US 8,022,251 and US 8,053,589 disclose one direct approach by using of Mannich adducts, which exploits the difference in volatility between distillable monomer and non-distillable by-products derived from the adduct. The adducts are derived from the reaction of cyanoacetate with stoichiometric quantities of specialised iminium-type methylene transfer (re)agents (CH₂=N⁺HR X-, wherein R = branched alkyl, X= sulfonate) that are prepared in an independent process from amines. The method produces stoichiometric quantities of voluminous ammonium salt by-products. Use of iminium salts in methylenation reactions has also been reported in Möhrle et al., Pharmazie, 1985, 40(10), 697-701, for vinyl diketone monomers.

In another direct approach, iminium or ammonium salts are instead used as organocatalysts, rather than reagents, for reaction between active methylene compounds and at least a stoichiometric quantity of a methylene diacetate, MeCOOCH₂OOCMe (an acylal, i.e., a compound with a *gem*-dicarboxy or *gem-*bis(acyloxy) moiety) that acts as a methylene transfer reagent, as disclosed in US 9,670,145. This method reports highest isolated monomer yields for CAs when the acylal is first prepared in a separate process.

Since all the aforementioned direct or indirect approaches rely on some nitrogen-based (N-based) species or catalyst, large excesses of acid must be applied at some point of whichever type of process to circumvent interaction with free basic amines that are highly destabilising towards the target electron-deficient olefin monomers. Such excesses create significant residue and large quantities of by-products, which must also be completely removed. Protonic acids are used generally to neutralise or acidify the reaction or process environment. Whereas electron deficient monomers are destabilised by bases, their polymerisation is inhibited by acids and since the Knoevenagel reaction is also known to be possible by Lewis acid catalysis, acid catalysed approaches have been described for the direct synthesis of CAs in three related patents deploying paraformaldehyde to transfer methylene groups.

In US 10,913,707 the relative conversion of cyanoacetate to cyanoacrylate in nitromethane-acetonitrile mixed solvents was only 14% after 6 hours at 90°C when a high loading (10 mol%) of a Lanthanide Lewis acid catalyst was employed. Conversion of starting materials to monomer was also low (<27%). A similar approach was described in US 10,889,542 and the result did not improve with extended reaction time. In both these latter cases the specific cyanoacetates were prepared *in situ* in a first step either by esterification of cyanoacetic acid or, by transesterification of ethyl cyanoacetate respectively using the same catalyst, before attempting monomer synthesis in a second step. In a third variation of the same reaction approach described in US 10,927,071, a pre-formed cyanoacetate was used instead and with reaction time up to 23 hours. In the latter case 20-30% of cyanoacetate starting material remained unconverted to monomer, and the monomer remained in solution. Such attempts at acid catalysed Knoevenagel synthesis of CAs so far show low efficiency even in solution. In none of the three aforementioned patents has the isolation of unprotected electron deficient monomers been demonstrated from solvent systems that are miscible with water that is produced in the reactions described.

In spite of the different approaches disclosed in prior art to prepare electron-deficient olefin monomers, there is a lack of an efficient process for preparing said monomers in stable environments from readily available starting materials under acid catalysis to circumvent the use of N-based catalysts and allow isolation of said reactive monomers directly from the reaction medium.

### Object of the invention

The object of the present invention is a process for preparing electron-deficient olefin monomers.

### Detailed Description of the Invention

The object of the present invention is a process for preparing electron-deficient olefin monomers, wherein an active methylene compound of general formula (III),

EWG-CH₂-Z¹ (III)

wherein the two electron withdrawing groups represented by EWG and Z¹, are selected from nitrile, carboxylic ester, and carboxylic acid functional groups, is reacted with either
a) a mixture of a source of formaldehyde and a carboxylic acid anhydride of general formula (IV), 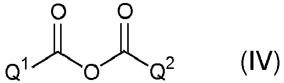
   wherein Q¹ and Q² are independently selected from linear and branched C₁-C₄ alkyl, optionally with H atoms substituted by F atoms, preferably are Me or Et, and more preferably are Me,
      in the presence of a catalyst system that comprises:
      i) at least one acid, or,
      ii) at least one acid and at least one co-catalyst,
   wherein the acid is selected from the group of Lewis and Brønsted acids, and the at least one co-catalyst is selected from the group of phenolic compounds, with one or more substituents, and quinone compounds, or
b) a methylene diester of general formula (V), 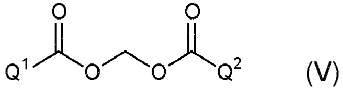 wherein Q1 and Q2 are independently selected from C₁-C₄ linear and branched alkyl groups,
   in the presence of a catalyst system that comprises a Brønsted acid and at least one co-catalyst selected from the group of phenolic compounds, with one or more substituents, and quinone compounds.

The authors of the present invention have developed a simple solution for preparing electron-deficient olefin monomers by using acid-based catalyst systems, preferably used in conjunction with promoters for Knoevenagel reactions, to convert active methylene compounds to said olefin monomers.

The authors of the present invention have developed a process for preparing electron-deficient olefin monomers that allows the direct preparation of the monomer without the need to include a step of thermal depolymerisation of a prepolymer. It is an efficient process for preparing said monomers in stable environments from readily available starting materials under acid catalysis to circumvent the use of N-based catalysts and allow isolation of said reactive monomers directly from the reaction medium. Since all CA processes generate large quantities of by-products from the various reagents that transfer or otherwise provide saturated methylene groups, atom-efficient processes are most desirable wherein by-products may be used to regenerate key reagents for the monomer forming reaction itself. Said electron-deficient olefin compounds are reactive monomers that find application in the field of fast-curing adhesives. These include cyanoacrylates, among other compounds.

The process of the invention allows the preparation of high purity monofunctional and polyfunctional electron-deficient olefin monomers independently of whether they are liquid or solid.

In the present description, as well as in the claims, the singular forms "a", "an" and "the" include the plural reference unless the context clearly indicates otherwise. The term "about" refers to a deviation of plus/minus 10%, preferably plus/minus 5%. The percentages are expressed in % by weight (wt%), unless stated the contrary. The ranges defined by the terms "between ... and ..." or by the terms "from ...to..." are meant to include also said stated endpoints thereof, and they also include any narrower sub-range.

The present invention comprises two main embodiments. One embodiment is referred to as a "three-component embodiment" and another, as a "two-component embodiment". For simplicity, however both embodiments comprise additional minor components that form the catalyst systems and stabilisers that are described separately for clarity.

### The three-component embodiment

The three-component embodiment is a preferred embodiment. So named because it comprises three groups of reactants, thus:
(i) active methylene compound,
(ii) a source of formaldehyde, and
(iii) a carboxylic acid anhydride.

In a preferred embodiment, the active methylene compound is a cyanoacetate, the source formaldehyde is 1,3,5-trioxane, the carboxylic acid anhydride is a symmetrical carboxylic acid anhydride, for example, acetic anhydride.

The reaction of the three-component embodiment is catalysed by a catalyst system that comprises at least one acid or, at least one acid and at least one co-catalyst.

The catalyst system allows the *in-situ* formation of an acylal by reaction of the source of formaldehyde and the carboxylic acid anhydride. The reaction between the acylal and the active methylene compound leads to the electron-deficient olefin monomer.

The three-component embodiment of the process of the invention for the above-mentioned preferred embodiment may be represented according to Scheme I for the specific case of the preparation of ethyl cyanoacrylate:

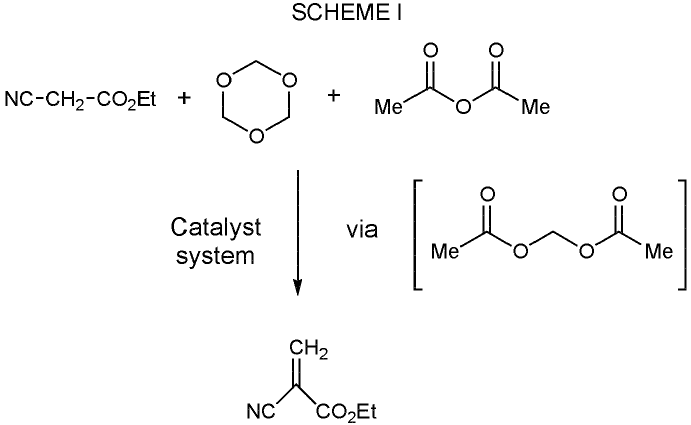

### The active methylene compound

The active methylene compounds of the process of the invention are the reactants that determine the type of electron-deficient olefin monomer that is produced.

In the present invention the active methylene compounds have general formula (III)

EWG-CH₂-Z¹ (III)

The preferred electron withdrawing functional groups in the active methylene compounds of the present invention represented by EWG and Z¹ in compounds of general formula (III) are selected from the group of nitrile (-CN), carboxylic ester (-CO₂R), and carboxylic acid (-CO₂H), more preferably from nitrile and carboxylic ester. In a preferred embodiment the active methylene compound comprises a nitrile and a carboxylic ester as electron withdrawing functional groups.

The preferred carboxylic ester groups (-CO₂R) are selected from those, wherein R is selected from linear C₁-C₁₈ alkyl, alkenyl or alkynyl, branched C₃-C₈ alkyl, cyclic or fused cycloaliphatic C₃-C₂₀, linear or branched C₁-C₄ alkoxyethyl, C₁-C₄ trialkylsiloxyethyl, tetrahydrofuryl, C₁-C₄ trialkyl silyl, -C₂H₄CO₂H, - (CH₂)ₙOC(O)CH₂CN where n= 2-4, - (CH₂)₂OC(O)CH=CH₂, and - (CH₂)₂OC(O)C(CH₃)=CH₂.

Active methylene compounds of general formula (III) are widely known. Many such compounds are commercially available, for example various alkyl cyanoacetates and 1,3-dicarbonyl compounds and the literature is replete with references to the preparation of others. The preparation of specialized cyanoacetates that may also be multifunctional or comprise mixed functionalities, is described in, for example, US 3,092,611, US 4,202,920, US 4,364876, US 4,528,357, US 5,140,084, US 5,175,337, US 6,096,848, US 6,271,410, US 6,281,310, US 7,262,313, EP-A-0 459 617, WO-A-2007/046647, WO-A-2008/069515, WO-A-2009/053482, Vijayalaskhmi et al., J. Adhesion Sci. Tech., 1990, 4(9), 733-750, Guseva et al., Izv. Akad. Nauk., Ser. Khim., 1993,3, 523-525, Kotzev et al., Die Makromol. Chem., 1980, 92(1421), 41-52, Blanco et al., Bull. Soc. Chim. Belg., 1989, 98(12), 923-929, Renner et al., J. Polym. Sci., Polym. Chem Ed., 1985, 23, 2341-2359, Senchenya et al., Izv. Akad. Nauk. Ser Khim., 1993, 42(5), 909-911, Urankar et al., J. Chem. Mater., 1997, 9, 2861-2868, Nicolas et al., Macromolecules, 2008, 41, 8418-8428, or Nakanishi et al., Chemistry Letts., 2007, 36(3), 452-453.

In some embodiments of the invention, the active methylene compounds of general formula (III) comprise a carboxylic acid group within an ester group, for example produced by the reaction of NCCH₂COO⁻ Na⁺ with bromoacetic acid.

In some embodiments of the invention, the active methylene compounds of general formula (III) comprise an electron withdrawing group that is the carboxylic acid group while the other electron withdrawing group is preferably selected from nitrile and carboxylic ester. Some of the latter compounds are commercially available, such as cyanoacetic acid. The preparation of others has been described by, for example, Levonis, et al., Aust. J. Chem., 2007, 60, 821-82, Xia et al., J. Chem. Res., 2005, 332-334, Kimura, M., Tetrahedron Lett., 2018, 59, 1295-1300, Mao et al., Eur. J. Org. Chem., 2020, (5), 525-538, or Bew et al., Chemistry, 2017, 23(19), 4557-4569.

Irrespective of type, one or more active methylene compound may be used in the three-component embodiment.

The concentration of active methylene compound(s) serves as a reference to the concentration of other components expressed in either mol% or stoichiometric/molar ratio relative to the compound(s) of general formula (III).

In an embodiment, the preferred active methylene compound is a cyanoacetate, or a mixture of cyanoacetates, or a mixture of cyanoacetate and cyanoacetic acid. In a more preferred embodiment, the preferred active methylene compound is a cyanoacetate.

### The source of formaldehyde

The three-component embodiment is contingent on the formation of the acylal, which requires the presence of a source of an aldehyde and a carboxylic acid anhydride. In the present invention, the source of the aldehyde is a source of formaldehyde.

The source of formaldehyde is selected from dry formaldehyde gas, paraformaldehyde, and 1,3,5-trioxane. The preferred source of formaldehyde is 1,3,5-trioxane, whether recrystallized or not, more preferably in a dry form achieved through any convenient means, such as recrystallisation from acetone, ethyl acetate, petroleum ethers, and mixtures thereof, or sublimation.

A preferred molar concentration of 1,3,5-trioxane relative to compound of general formula (III) is 1.25:1, more preferably 1.1:1, and yet more preferably 1:1.

### The carboxylic acid anhydride

As exposed above, the three-component embodiment in the process of the invention requires the presence of a source of formaldehyde and a carboxylic acid anhydride to obtain *in situ* the acylal, which, under the Knoevenagel reaction conditions, further reacts with the active methylene compound of formula (III) to obtain the electron-deficient olefin monomers.

In the process of the invention are used carboxylic acid anhydrides of general formula (IV), 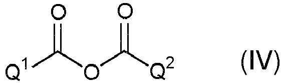 wherein Q¹ and Q² are independently selected from linear and branched C₁-C₄ alkyl, optionally with H atoms substituted by F atoms, preferably are Me or Et, and more preferably are Me.

In a preferred embodiment, Q¹ and Q² are the same group, i.e., the carboxylic acid anhydride is symmetrical. In a more preferred embodiment, the carboxylic acid anhydride is selected from acetic anhydride and propionic anhydride. In a yet more preferred embodiment, the carboxylic acid anhydride is acetic anhydride.

Triflic anhydride may also be used in the present invention and promotes faster reaction still than acetic anhydride. However acetic anhydride is more convenient to handle.

As is well known in the art, carboxylic acid anhydrides may be readily prepared from carboxylic acids, for example by the mild methods described in Kim et al., Synthetic Comm., 2001, 31(3), 395-399, and references cited therein.

In the preferred embodiment, wherein acetic anhydride is used, acetic acid is generated as a by-product in the process of the invention, and may thus be re-used to reproduce acetic anhydride for use again in said process by using such methods.

The process of the invention is atom-efficient in this regard, unlike the indirect or direct methods employing paraformaldehyde or direct methods producing salts, all of which generate residue as waste.

In the process of the invention, the carboxylic acid anhydride for the three-component embodiment is employed usually in the molar concentration range ratio 1:5, preferably in the molar concentration range ratio 1:3, more preferably in the molar concentration range ratio 1:2.5, and yet more preferably in the ratio 1:2, where the ratios refer to active methylene compound of general formula (III) to acid anhydride compound of general formula (IV), that is the ratio of (III) to (IV).

### The catalyst system

In the process of the invention, the reaction of the three-component embodiment is catalysed by a catalyst system that comprises at least one acid or at least one acid and at least one co-catalyst selected from the group of phenolic compounds, with one or more substituents, and quinone compounds.

The catalyst system also functions as a stabiliser system although additional polymerisation inhibitors may also be incorporated.

An objective of the present invention is the direct preparation of electron-deficient olefinic monomers by reaction between active methylene compounds and acylals, either formed *in situ* or preformed, with novel efficient catalyst systems that are acid based.

Promotion of reaction by *in situ* formation of acylals contributes to reaction efficiency as does the use of catalysts that play more than one role. Situations may arise wherein a catalyst known to facilitate acylal formation is not an efficient catalyst for Knoevenagel reaction or *vice versa.* For example, Brønsted acids readily catalyse acylal formation, but are not catalysts of choice for independent Knoevenagel reactions.

On the other hand, compounds such as hydroquinone used in concentrations as low as 0.05 mol% can catalyse the nucleophilic addition of active methylene compounds activated by benzoquinone at similar concentrations to substituted aldehydes in Knoevenagel reactions as indicated in, for example, Takakura et al., Org. Biomol. Chem., 2020, 18, 6594-6597, but neither hydroquinone or benzoquinone independently catalyse acylal formation.

In the present invention acid-based catalyst systems produce electron deficient olefin monomers by facilitating reaction between acylals and active methylene compounds whether or not the components comprising the catalyst system independently catalyse either acylal formation, or, Knoevenagel reaction. The catalyst systems, suitable to be used in the process of the invention, provide multiple means of activation of all reactants and are preferably provided through the combination of acid catalysts with co-catalysts especially when either acid catalyst or co-catalyst cannot independently influence both acylal formation and Knoevenagel reaction. Catalyst systems comprising such combinations also facilitate direct reaction between active methylene compounds and pre-formed acylals. It has been surprisingly found that effective co-catalysts for Lewis or Brønsted acid catalysts are selected from phenolic compounds and quinone compounds.

The catalyst systems described herein thus comprise either specific Lewis acids, combinations of Lewis acids and Brønsted acids, combinations of Lewis acids or Brønsted acids with aforementioned co-catalysts, or combinations of Lewis acids and Brønsted acids and said co-catalysts.

In the three-component embodiment of the present invention, the catalyst system is selected from:
1) a Lewis acid, preferably a post-transition metal Lewis acid,
2) a combination of a Lewis acid, preferably a post-transition metal Lewis acid, and a Brønsted acid,
3) a combination of a Lewis acid, preferably a post-transition metal Lewis acid, and a co-catalyst selected from phenolic compounds, with one or more substituents, quinone compounds, and mixtures thereof,
4) a combination of a Brønsted acid and a co-catalyst selected from phenolic compounds, with one or more substituents, quinone compounds, and mixtures thereof, and
5) a combination of a Lewis acid, preferably a post-transition metal Lewis acid, and a Brønsted acid and a co-catalyst selected from phenolic compounds, with one or more substituents, quinone compounds, and mixtures thereof.

Preferably the co-catalyst is selected from phenol, hydroquinone, p-methoxyphenol, p-trifluoromethyl phenol, 1,4-benzoquinone, 1,4-naphthoquinone, and mixtures thereof. More preferably, the co-catalyst is hydroquinone.

In a preferred three-component embodiment of the present invention, the catalyst system catalyses a reaction between a cyanoacetate active methylene compound and methylene diacetate (an acylal), produced *in situ,* by acid catalysed reaction between acetic anhydride and a source of formaldehyde, preferably 1,3,5-trioxane.

The use of said catalyst systems in conjunction with a carboxylic acid anhydride has not been previously described for the direct synthesis of cyanoacrylates from cyanoacetates.

The acid catalyst system present in the process of the invention also stabilises against cyanoacrylate polymerisation.

In a most preferred three-component embodiment of the present invention, the catalyst system comprises a combination of Lewis acid, preferably a post-transition metal Lewis acid, a Brønsted acid and a co-catalyst selected from phenolic compounds, with one or more substituents, quinone compounds, and mixtures thereof, preferably selected from phenol, hydroquinone, *p*-methoxyphenol, *p*-trifluoromethyl phenol, 1,4-benzoquinone, 1,4-naphthoquinone, and mixtures thereof.

In a highly preferred embodiment of the process of the invention, it is used an alkyl cyanoacetate as active methylene compound, a source of formaldehyde, preferably 1,3,5-trioxane, acetic anhydride, and the catalyst system comprises a combination of Lewis acid, preferably a post-transition metal Lewis acid, a Bronsted acid and a co-catalyst selected from phenolic compounds, with one or more substituents, quinone compounds, and mixtures thereof, preferably selected from phenol, hydroquinone, *p*-methoxyphenol, *p*-trifluoromethyl phenol, 1,4-benzoquinone, 1,4-naphthoquinone, and mixtures thereof.

Various Lewis and Bronsted acids or their mixtures may be used in the catalyst systems in the three-component embodiment of the process of the invention.

Lewis acids suitable to be used in the process of the invention are selected from transition metals halides, post-transition metal-based halides, preferably trihalides, alkali earth metal-based perchlorates, such as Mg(ClO₄)₂, and Lanthanide metal based, such as Yb(OTf)₃. In a preferred embodiment, Lewis acids are selected from the group of post-transition metals, more preferably from indium (III) trihalide and gallium (III) trihalide, preferably trichlorides, and most preferably the Lewis acid is InCl₃.

In the three-component embodiment, the Lewis acid catalyst may be used in the concentration range comprised between 0.05 mol% and 10 mol% relative to active methylene compound of general formula (III). Preferably it is used in the range comprised between 0.1 mol% and 5 mol%, and most preferably in the range comprised between 0.5 mol% and 1.5 mol%. The use of such low catalyst loadings further adds convenience in processing, since catalysts are not required to be separated from reaction mixtures for subsequent distillations, and reactions may be conducted in continuous mode.

Various Bronsted acids are suitable to be used in the process of the invention. Preferably they are selected from *p*-toluene sulfonic acid, benzene sulfonic acid, and aralkylsulfonic acids. Most preferably the Brønsted acid is *p*-toluene sulfonic acid, whether anhydrous form or as monohydrate.

In the three-component embodiment, the Bronsted acid catalyst may be used in the concentration range comprised between 2.5 mol% and 15 mol%, relative to active methylene compound of general formula (III), preferably it is used in the range comprised between 4 mol% and 6 mol%.

As above-mentioned, in most cases combination of a Lewis and/or a Bronsted acid with at least one co-catalyst is preferred.

The co-catalysts are preferably used in the concentration range comprised between 0.01 mol% and 10 mol%, relative to active methylene compound of general formula (III), more preferably between 0.01 mol% and 6 mol%.

In combined catalyst systems, Lewis acid and Bronsted acids are preferably used in concentrations comprised between 0.5 mol% and 1 mol% and between 2 mol% and 8 mol%, respectively, relative to active methylene compound of general formula (III). Lewis acids and co-catalysts are preferably used in concentrations comprised between 0.5 mol% and 1mol% and between 0.02 mol% and 8 mol% respectively, relative to active methylene compound of general formula (III), Brønsted acid and co-catalysts are preferably used in concentrations comprised between 1 mol% and 5 mol% and between 0.02 mol% and 8 mol%, respectively, relative to active methylene compound of general formula (III), and Lewis acids and Brønsted acids and co-catalysts are preferably used in concentrations comprised between 0.5 mol% and 1 mol% and between 2 mol% and 8 mol% and between 0.02 mol% and 8 mol%, respectively, relative to active methylene compound of general formula (III).

### Stabilisers

Electron-deficient olefin monomers, such as CAs, are readily polymerised by electron rich species. Since they are vinyl monomers, CAs are also susceptible to polymerisation by free radicals even if this is a less preferred mechanism. Synthesis and manipulation of these reactive monomers in all process steps necessitates special attention to stabilisation in both the liquid phase and vapour phase. In turn this requires use of non-volatile and volatile stabilisers to prevent polymerisation by any mechanism.

Additionally, as well-known by the skilled person in the art, equipment that is in direct contact with the monomers must be passivated, for example glassware or stainless-steel components are pre-washed with strong Bronsted acids such as sulfuric acid, thoroughly rinsed with distilled water and dried.

Liquid CA monomers isolated by distillation are usually collected onto a quantity of combined acidic and free radical stabilisers pre-determined according to the expected volume of monomer to be collected. Whereas the catalyst system of the inventive three-component embodiment inherently possesses stabilisation properties that inhibit polymerisation of highly reactive monomers, additional stabilisers may optionally be employed in the reaction and distillation processes as well as in final distillates from the reaction.

In an embodiment, the process of the invention comprises the use of stabilisers selected from acid stabilisers and radical stabilisers.

Volatile acid stabilisers are well known in the prior art to curtail polymerisation in the vapour trail in distillation processes with electron deficient monomers. Lewis or Brønsted acids such as boron trifluoride etherate, SO₂, trifluoracetic acid, most preferably boron trifluoride etherate, may be used in concentration ranges comprised between 0 wt% and 0.05 wt% in bulk reaction milieux and distillation pots, and comprised between 0.0005 wt% and 0.005 wt% in monomeric distillates.

Non-volatile free radical stabilisers such as, for example, 2,2'-methylenebis-(6-tert-buthyl-p-cresol) (Stabilizer "2246"), pentaerythritol tetrakis[3-[3,5-di-tert-butyl-4-hydroxyphenyl]propionate (Irganox^{®} 1010) may be used in concentration ranges comprised between 0.01 wt% and 3.0 wt%, preferably between 0.01 wt% and 1.5 wt%, in bulk reaction milieux and distillation pots.

Volatile free radical stabilisers may also be used in the process of the invention. Said stabilisers include, for example, hydroquinone (HQ), 4-methoxyphenol (MeHQ), butylated hydroxyanisole (BHA), and butylated hydroxytoluene (BHT), and may be used in concentration ranges comprised between 0.01 wt% and 3.0 wt%, preferably between 0.01 wt% and 1.5 wt%, in bulk reaction milieux and distillation pots, and comprised between 0.1 wt% and 0.5 wt% in monomeric distillates.

### Solvent

The three-component embodiment may be conducted solventless or in the presence of solvents such as, for example, toluene, *p*-xylene, or 1,4-dioxane.

Hydrocarbon solvents also provide a useful means of pre-drying reaction components, if necessary, by formation of azeotropes with any water that may be present and use of Dean-Stark equipment as well known to those skilled in the art.

If a solvent Is used, typically, the reaction mixture is diluted by 1.5 to 10-fold by volume, preferably by 2-fold by volume.

### The three-component process

Typically, the reaction mixtures are continuously agitated by any appropriate means such as stirring and/or shearing in batch reactions, or, through use of static or dynamic mixers or spinning discs in continuous or flow reactors.

In batch reactors, the reactions are generally conducted in a timeframe of 1 to 10 hours depending on the reactivity of the substrates, the catalyst system and the temperature of reaction.

Typically, preferred reaction temperatures are in the range comprised between 60°C and 140°C, for reaction time in a range comprised between 0.5 h and 10 h, preferably between 100°C and 130°C for reaction time between 1 h and 5 h, and more preferably between 115°C and 125°C for reaction time between 1 h and 2 h.

In a preferred embodiment, the reactions are conducted within 2 h at 120°C employing a catalyst system comprising InCl₃ at 1 mol%, p-toluene sulfonic acid at 5 mol% and hydroquinone at 5 mol%.

By scaling the batch process, the skilled person may adjust the reaction conditions using the common general knowledge.

The parameters required to optimise continuous reaction depend on the type of reactor employed and are determined in ways known to those skilled in the art by tracking conversion to product through sampling as a function of time and systematic variation of reaction component flow rates and agitation speeds. In this regard the use of benchtop ¹H-NMR (Spinsolve 60MHz, Magritek Ltd.) is particularly useful for fast feedback. A preferred continuous reactor is the SpinPro rector (Flowid bv) because of its capability to process inhomogeneous mixtures with exceptional mixing.

### The two-component embodiment

The two-component embodiment comprises two groups of reactants, thus:
(i) active methylene compound, and
(ii) a methylene diester (acylal).

In a preferred embodiment, the active methylene compound is a cyanoacetate, and the methylene diester is selected from methylene diacetates, for example, methylene diacetate. The reaction of the two-component embodiment is catalysed by a catalyst system that comprises a Bronsted acid and at least one co-catalyst selected from the group of phenolic compounds, with one or more substituents, and quinone compounds.

The catalyst system also functions as a stabiliser system although additional polymerisation inhibitors may also be incorporated.

The two-component embodiment of the process of the invention for the specific above-mentioned preferred embodiment may be represented according to Scheme II for the specific preparation of ethyl cyanoacrylate:

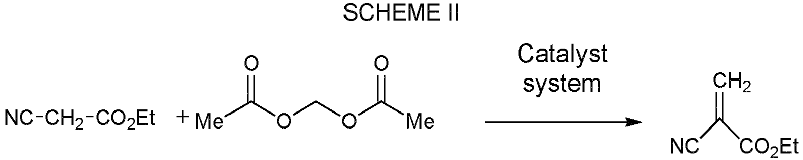

### The active methylene compound

The active methylene compounds used in the three-component embodiment also apply to the two-component embodiment.

### The acylal compound

The acylal compound is a compound with a *gem*-dicarboxy or *gem*-bis(acyloxy) moiety, such as, for example, methylene diacetate.

Whereas in the three-component embodiment of the present invention, an acylal is produced *in situ,* in the two-component embodiment, the acylal is provided after its pre-formation in an independent reaction. As with the three-component embodiment, a means to activate the active methylene compound with a suitable catalyst system to facilitate its reaction with the acylal is also required.

The terms acylal, methylene diester, methylene diacetate, 1,1-geminal diacetate are used interchangeably to describe compounds of general formula (V): 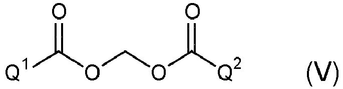 wherein Q¹ and Q² are independently selected from C₁-C₄ linear and branched alkyl groups, and said terms also refer to acylals, wherein the central methylene group is substituted as shown in the general formula (VI): 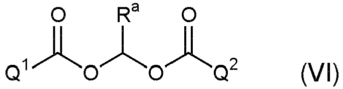 wherein R^{a} selected from alkyl, aryl, and aralkyl group.

Methylene diesters of formula (VI) that bear a substituent on the central methylene group, result from reaction of carboxylic acid anhydrides with substituted aldehydes have been extensively reviewed in Sydnes et al., PINSA, 2002, 68(2), 141-174.

Acylals are usually prepared by the acid catalysed reaction of a carboxylic acid anhydride with an aldehyde, however they may also be prepared by transfer of a methylene group from donors such as methylene dihalides (e.g., dichloro- or diiodomethane) to carboxylates.

Methylene diacetates comprising a central group that is unsubstituted (thus, -OCH₂O-) result from reaction of carboxylic acid anhydrides with linear or cyclic sources of formaldehyde such as paraformaldehyde or 1,3,5-trioxane respectively, in the presence of acid or metal-based catalysts and are also described in, for example, US 3, 927,078, Hoffmann et al., Catal. Sci. Technol., 2018, 8, 5594-5603, and Tomiška *et al., Angew. Chem. Int. Edn.,* 1962, 1(4), 211. Preferred acylals used in the two-component embodiment of the present invention exclusively comprise an unsubstituted central methylene group (-OCH₂O-). The groups Q¹ and Q² are preferably equivalent in the acylals of choice and selected from C₁-C₄ linear and branched alkyl groups.

Acylals derived from propionic anhydride are disclosed in Dalpozzo et al., ARKIVOC, 2006, (vi), 181-189.

Acylals derived from butyric, isobutyric and pivalic anhydrides have been described in Sumida et al., Synletts, 2001, 1921-1922, and Samitha et al., Tetrahedron, 2003, 59, 9571-9576. The most preferred methylene diester involved in either embodiment of the present invention is methylene diacetate in which Q¹ and Q² in general formula (V) are methyl groups.

A wide variety of protic acid, Lewis acid and heterogenous catalysts have been described in the literature for the independent synthesis of acylals, such as, for example, AlCl₃, Amberlyst, Envirocatsw, montmorillonite, zeolites, Nafion-H, Dowex^{®}50WX4, Caro's acid, Wells-Dawson heteropolyacid, Mo/TiO2-ZrO₂ solid acid, P₂O₅/SiO₂, P₂O₅/montmorillonite, FeSO₄, FeCl₃, AlCl₃, TMSCI-Nal, Sc(OTf)₃, I₂, NBS, PCl₃, H₂SO₄, Cu(OTf)₂, LiBF₄, H₂NSO₃H, BF₃, (NH₄)₂Ce(NO₃)₆, LiOTf, Zn(BF₄)₂, AlPW₁₂O₄₀, ZrCl₄, Bi(NO₃)₃.5H₂O, Bi(CF₃SO3).4H₂O, zirconium sulfohexyl phosphonate, sulphated zirconia, poly(N,N - dibromo-N-ethyl-benzene-1,3-disulfonamide) [PBBS], *N*,*N*,*N*,*N*-tetrabromobenzene-1,3-disulfonamide [TBBDA], tetrabromo-2,5-cyclohexadienone (TABCO), tetrabutylammonium tribromide (TBATB), saccharin sulfonic acid, zirconium hydrogen sulfate, Fe₂(SO₄)₃·xH2O, alum [KAl(SO₄)₂.12H₂O], sulfated zirconia, KHCO₃, sulfuric acid, H₂SO₄-silica, HClO₄-SiO₂, lithium perchlorate, zinc(II) perchlorate, [Hmim][HSO₄] [Brønsted acidic ionic liquid], Al(HSO₄)₃. Preferred catalysts for the formation of acylals are selected from those known to function under mild conditions in independent reactions, such as the alkali metal based Lewis acid LiBr described in Sampath Kumar et al., J. Chemical Research (S), 2000, 86-87, the alkali earth based Lewis acid Mg(ClO₄)₂ described in Yang, in J. Chemical Research, 2006, 199-202, Lanthanide based Lewis acids such as Yb(OTf)₃ and Er(OTf)₃, described in Dalpozzo *et al., op. cit.,* transition metal Lewis acid catalysts such as FeCl₃ described in the review Rezayati et al., Research on Chemical Intermediates, 2020, DOI: 10.1007/s11164-020-04176-x, and post-transition metal Lewis acid catalysts such as indium and gallium trihalides described in Yin et al., Tetrahedron, 2007, 48, 3119-3122, and Kumar et al., ARKIVOC, 2007, 27-33.

### The catalyst system

In the two-component embodiment of the process of the invention, the catalyst system allows the reaction between the acylal and the active methylene compound leading to the electron-deficient olefin monomer.

The catalyst system comprises a Brønsted acid and at least one co-catalyst.

Various Brønsted acids are suitable to be used in the process of the invention. Preferably they are selected from p-toluene sulfonic acid, benzene sulfonic acid, and aralkylsulfonic acids. Most preferably the Brønsted acid is *p*-toluene sulfonic acid, whether anhydrous form or as monohydrate.

The co-catalyst is selected from phenolic compounds with one or more substituents, and quinone compounds. Preferably the co-catalyst is selected from phenol, hydroquinone, *p-*methoxyphenol, *p*-trifluoromethyl phenol, 1,4-benzoquinone, 1,4-naphthoquinone, and mixtures thereof. More preferably the co-catalyst is hydroquinone.

The catalyst and co-catalysts present are also polymerisation stabilisers. The combined use of a Bronsted acid catalyst with hydroquinone as a co-catalyst has not been previously described for the direct synthesis of electron deficient olefin monomers such as cyanoacrylates.

The preferred Brønsted acid catalyst and co-catalyst concentrations are comprised between 2 mol% and 5 mol% of Brønsted acid and between 0.02 mol% and 8 mol% of co-catalyst, relative to active methylene compound of general formula (III).

The conditions applied in the three-component embodiment also apply to the two-component embodiment. The preferred ratio of active methylene compound to acylal is 1:1.

The two-component embodiment may also optionally deploy additional stabilisers as described for the three-component embodiment.

### Industrial applicability

The process of the present invention has advantages over the prior art indirect and direct methods aside from circumvention of the aggressive cracking needed by the former.

Firstly, use of acidic catalysis provides safeguards in processes especially when high levels of conversion of starting materials to high concentrations of reactive monomers are achieved in relatively fast and even solventless reactions as in the present invention. The inventive catalyst system also comprises compounds known to function as free radical stabilisers that are co-catalysts. Acids inhibit polymerisation which is especially desirable in methods wherein monomer is unprotected in reaction milieux and in subsequent process steps. Avoidance of the use of N-based catalysts is desirable since inadvertent release of basic compounds from ammonium or iminium salts under process conditions presents a risk, even in the presence of excesses of protonic acids. Even trace levels of basic compounds are potent initiators for the polymerisation of such monomers. Hofmann elimination-, Knoevenagel-, and Mannich-reaction types are well-known wherein N-based salts exist in some equilibrium with parent free amines as reviewed or reported in, for example, van Beurden *et al.,* op. cit., and Dalessandro, et al., Phys. Chem., B, 2017, 121(20), 5300-5307, and such reactions form the basis of several of the previously mentioned direct processes.

Preferential use of suitable acid catalysts as opposed to N-based catalysts in the present invention is not the only advantage, since the deployment and mode of operation using some specific Lewis acid catalysts in unpromoted Knoevenagel reactions has not led to an efficient process for isolating directly synthesised CA monomers in prior art. The present invention uses lower catalyst loadings than used in any previously reported process, which reduces cost, reaction residue and waste, improves reaction efficiency and selectivity and facilitates uninterrupted processing steps including monomer isolation. Still further reaction by-products may be re-used in the inventive processes to produce promoters or reagents for the monomer forming reaction itself.

The process of the invention is applicable to industry for the facile one-pot preparation of reactive monomers for use in adhesives, sealants and coatings. The methods described deploy low loadings of simple commercially available catalysts and co-catalysts that also assist stabilisation of the reaction even under concentrated conditions. Standard methods are known that convert carboxylic acids to their anhydrides, and the latter have been shown to be promoters for the monomer forming reaction itself or are known reactants that in turn can be used to pre-form acylals that can be used in the monomer forming reaction.

The product output from either embodiment of the invention from either batch or flow reactors may be directly fed into continuous vacuum evaporation or distillation systems. Electron deficient monomers are isolated by fractional distillation preferably employing vapour trail stabilisation using volatile free radical and Lewis acids stabilisers and collecting monomer onto stabilisers at previously indicated concentration levels. Volatile reaction components such as solvents and/or excess reagents if present, and by-products such as carboxylic acids are easily removed by evaporation and fractional distillations since their boiling points are much lower than target monomers. All evaporation or distillation equipment used for condensing vapour and collecting reactive monomer is usually passivated before use by pre-washing with, for example, 10% sulfuric acid, rinsing with distilled water and thoroughly dried before use.

The process demonstrates isolation of pure highly reactive monomer by direct distillation from the reaction medium when starting materials are converted without the need for any intermediate processing steps. Circumventing the need to interrupt processing from synthesis to distillation lends the inventive methods to batch or continuous processing. The method is high yielding even for the most reactive ethyl cyanoacrylate monomer, with or without the use of additional solvents. The method does not produce large quantities of residue or waste, such as crack-residue or intractable masses of salts that must be disposed of.

The invention comprises the following embodiments:
1.- A process for preparing electron-deficient olefin monomers, characterized in that an active methylene compound of general formula (III),

   EWG-CH₂-Z¹ (III)

   wherein the two electron withdrawing groups represented by EWG and Z¹, are selected from nitrile, carboxylic ester, and carboxylic acid functional groups,
   is reacted with either
   a) a mixture of a source of formaldehyde and a carboxylic acid anhydride of general formula (IV), 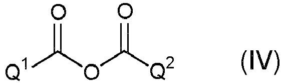 wherein Q¹ and Q² are independently selected from linear and branched C₁-C₄ alkyl, optionally with H atoms substituted by F atoms, preferably are Me or Et, and more preferably are Me,
      in the presence of a catalyst system that comprises:
      i) at least one acid, or,
      ii) at least one acid and at least one co-catalyst,
         wherein the acid is selected from the group of Lewis and Bronsted acids, and the at least one co-catalyst is selected from the group of phenolic compounds, with one or more substituents, and quinone compounds, or
   b) a methylene diester of general formula (V), 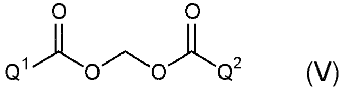 wherein Q1 and Q2 are independently selected from C₁-C₄ linear and branched alkyl groups,
      in the presence of a catalyst system that comprises a Brønsted acid and at least one co-catalyst selected from the group of phenolic compounds, with one or more substituents, and quinone compounds.
2.- The process according to embodiment 1, characterized in that the active methylene compound of general formula (III),

   EWG-CH₂-Z¹ (III)

   wherein the two electron withdrawing groups represented by EWG and Z¹, are selected
   from nitrile, carboxylic ester, and carboxylic acid functional groups, is reacted with a mixture of a source of formaldehyde and a carboxylic acid anhydride of general formula (IV), 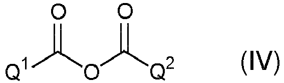 wherein Q¹ and Q² are independently selected from linear and branched C₁-C₄ alkyl, optionally with H atoms substituted by F atoms, preferably are Me or Et, and more preferably are Me,

   in the presence of a catalyst system that comprises:
      i) at least one acid, or,
      ii) at least one acid and at least one co-catalyst,
   wherein the acid is selected from the group of Lewis and Brønsted acids, and the at least one co-catalyst is selected from the group of phenolic compounds, with one or more substituents, and quinone compounds.
3.- The process according to embodiments 1 or 2, characterized in that the active methylene compound of formula (III) comprises a nitrile and a carboxylic ester as electron withdrawing functional groups.
4.- The process according to any one of embodiments 1 to 3, characterized in that the carboxylic ester groups (-CO₂R) are selected from those, wherein R is selected from linear C₁-C₁₈ alkyl, alkenyl or alkynyl, branched C₃-C₈ alkyl, cyclic or fused cycloaliphatic C₃-C₂₀, linear or branched C₁-C₄ alkoxyethyl, C₁-C₄ trialkylsiloxyethyl, tetrahydrofuryl, C₁-C₄ trialkyl silyl, - C₂H₄CO₂H, - (CH₂)ₙOC(O)CH₂CN where n= 2-4, - (CH₂)₂OC(O)CH=CH₂, and - (CH₂)₂OC(O)C(CH₃)=CH₂.
5.- The process according to any one of embodiments 1 to 4, characterized in that the active methylene compound is a cyanoacetate, or a mixture of cyanoacetates, or a mixture of cyanoacetate and cyanoacetic acid, preferably it is a cyanoacetate.
6.- The process according to any one of embodiments 1 to 5, characterized in that the source of formaldehyde is selected from dry formaldehyde gas, paraformaldehyde, and 1,3,5-trioxane, preferably it is 1,3,5-trioxane.
7.- The process according to any one of embodiments 1 to 6, characterized in that the molar concentration of 1,3,5-trioxane relative to compound of general formula (III) is 1.25:1, preferably 1.1:1, and more preferably 1:1.
8.- The process according to any one of embodiments 1 to 7, characterized in that it is used the carboxylic acid anhydride of formula (IV) wherein Q¹ and Q² are the same group.
9.- The process according to embodiment 8, characterized in that the carboxylic acid anhydride of formula (IV) is selected from acetic anhydride and propionic anhydride, preferably the carboxylic acid anhydride is acetic anhydride.
10.- The process according to any one of embodiments 1 to 9, characterized in that the carboxylic acid anhydride is employed in the molar concentration range ratio 1:5, preferably in the molar concentration range ratio 1:3, more preferably in the molar concentration range ratio 1:2.5, and yet more preferably in the ratio 1:2, where the ratios refer to active methylene compound of general formula (III) to acid anhydride compound of general formula (IV).
11.- The process according to any one of embodiments 1 to 10, characterized in that the catalyst system is selected from a Lewis acid, preferably a post-transition metal Lewis acid; a combination of a Lewis acid, preferably a post-transition metal Lewis acid, and a Bronsted acid; a combination of a Lewis acid, preferably a post-transition metal Lewis acid, and a co-catalyst selected from phenolic compounds, with one or more substituents, quinone compounds, and mixtures thereof; a combination of a Brønsted acid and a co-catalyst selected from phenolic compounds, with one or more substituents, quinone compounds, and mixtures thereof, and a combination of a Lewis acid, preferably a post-transition metal Lewis acid, and a Brønsted acid and a co-catalyst selected from phenolic compounds, with one or more substituents, quinone compounds, and mixtures thereof, preferably it is a combination of Lewis acid, preferably a post-transition metal Lewis acid, a Bronsted acid and a co-catalyst selected from phenolic compounds, with one or more substituents, quinone compounds, and mixtures thereof.
12.- The process according to any one of embodiments 1 to 11, characterized in that the co-catalyst is selected from phenol, hydroquinone, *p*-methoxyphenol, *p*-trifluoromethyl phenol, 1,4-benzoquinone, 1,4-naphthoquinone, and mixtures thereof, preferably, it is hydroquinone.
13.- The process according to any one of embodiments 1 to 12, characterized in that it is used an alkyl cyanoacetate as active methylene compound, a source of formaldehyde, preferably 1,3,5-trioxane, acetic anhydride, and the catalyst system comprises a combination of Lewis acid, preferably a post-transition metal Lewis acid, a Brønsted acid and a co-catalyst selected from phenolic compounds, with one or more substituents, quinone compounds, and mixtures thereof, preferably selected from phenol, hydroquinone, *p*-methoxyphenol, *p*-trifluoromethyl phenol, 1,4-benzoquinone, 1,4-naphthoquinone, and mixtures thereof.
14.- The process according to any one of embodiments 1 to 13, characterized in that the Lewis acid is selected from transition metals halides, post-transition metal-based halides, preferably trihalides, alkali earth metal-based perchlorates, and Lanthanide metal based, preferably selected from the group of post-transition metals, more preferably from indium (III) trihalide and gallium (III) trihalide, preferably trichlorides, and yet more preferably the Lewis acid is InCl₃.
15.- The process according to any one of embodiments 1 to 14, characterized in that the Lewis acid catalyst is used in the concentration range comprised between 0.05 mol% and 10 mol% relative to active methylene compound of general formula (III), preferably it is used in the range comprised between 0.1 mol% and 5 mol%, and more preferably in the range comprised between 0.5 mol% and 1.5 mol%.
16.- The process according to any one of embodiments 1 to 15, characterized in that the Brønsted acid is selected from *p*-toluene sulfonic acid, benzene sulfonic acid, and aralkylsulfonic acids, preferably it is *p*-toluene sulfonic acid, whether anhydrous form or as monohydrate.
17.- The process according to any one of embodiments 1 to 16, characterized in that the Brønsted acid catalyst is in the concentration range comprised between 2.5 mol% and 15 mol% relative to active methylene compound of general formula (III), preferably it is used in the range comprised between 4 mol% and 6 mol%.
18.- The process according to any one of embodiments 1 to 17, characterized in that the co-catalyst is used in the concentration range comprised between 0.01 mol% and 10 mol%, relative to active methylene compound of general formula (III), more preferably between 0.01 mol% and 6 mol%.
19.- The process according to embodiments 1 or 2, characterized in that in combined catalyst systems, Lewis acid and Bronsted acids are used in concentrations comprised between 0.5 mol% and 1 mol% and between 2 mol% and 8 mol%, respectively, relative to active methylene compound of general formula (III); Lewis acids and co-catalysts are used in concentrations comprised between 0.5 mol% and 1mol% and between 0.02 mol% and 8 mol% respectively, relative to active methylene compound of general formula (III); Bronsted acid and co-catalysts are used in concentrations comprised between 1 mol% and 5 mol% and between 0.02 mol% and 8 mol%, respectively, relative to active methylene compound of general formula (III); and Lewis acids and Brønsted acids and co-catalysts are used in concentrations comprised between 0.5 mol% and 1 mol% and between 2 mol% and 8 mol% and between 0.02 mol% and 8 mol%, respectively, relative to active methylene compound of general formula (III).
20.- The process according to any one of embodiments 1 to 19, characterized in that it comprises the use of stabilisers selected from acid stabilisers, preferably selected from boron trifluoride etherate, SO₂, trifluoracetic acid, more preferably boron trifluoride etherate; and radical stabilisers, preferably selected from 2,2'-methylenebis-(6-*tert*-buthyl-*p*-cresol), pentaerythritol tetrakis[3-[3,5-di-*tert*-butyl-4-hydroxyphenyl]propionate hydroquinone, 4-methoxyphenol, butylated hydroxyanisole, and butylated hydroxytoluene.
21.- The process according to embodiment 1, characterized in that the active methylene compound of general formula (III),

   EWG-CH₂-Z¹ (III)

   wherein the two electron withdrawing groups represented by EWG and Z¹, are selected from nitrile, carboxylic ester, and carboxylic acid functional groups,
   is reacted with a methylene diester of general formula (V), 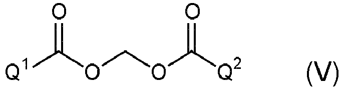 wherein Q1 and Q2 are independently selected from C₁-C₄ linear and branched alkyl groups,
   in the presence of a catalyst system that comprises a Bronsted acid and at least one co-catalyst selected from the group of phenolic compounds, with one or more substituents, and quinone compounds.
22.- The process according to embodiment 21, characterized in that it is used an active methylene compound of general formula (III) as defined in any one of embodiments 3 to 5.
23.- The process according to embodiments 21 or 22, characterized in that the methylene diester is methylene diacetate.
24.- The process according to any one of embodiments 21 to 23, characterized in that it is used a Brønsted acid as defined in embodiment 16.
25.- The process according to any one of embodiments 21 to 24, characterized in that it is used a co-catalyst as defined in embodiment 12.
26.- The process according to any one of embodiments 21 to 25, characterized in that the Brønsted acid catalyst and co-catalyst concentrations are comprised between 2 mol% and 5 mol% and between 0.02 mol% and 8 mol%, respectively, relative to active methylene compound of general formula (III).
27.- The process according to any one of embodiments 21 to 26, characterized in that the ratio of active methylene compound of formula (III) to acylal of formula (V) is 1:1.
28.- The process according to any one of embodiments 21 to 26, characterized in that it comprises stabilisers as defined in embodiment 20.

Next, several examples are provided which are illustrative of the invention but which are not limitative thereof.

### Examples

### Methodology

The process of the invention in either embodiment was conducted in air, although an inert gas atmosphere such as provided by nitrogen or argon gas could be used.

The reagents and catalyst were simply mixed together in one-pot and the reaction was conducted at temperature and for specific times.

Reasonable standard measures common to those skilled in the art were taken to avoid excessive moisture originating from raw materials or the ambience of the reaction in the three-component embodiment of the invention since acid anhydrides are moisture sensitive, however the use of strictly anhydrous conditions in any embodiment is not necessary.

Lewis acids such as InCl₃ are deliquescent solids and were used as received.

Brønsted acid *p*-toluene sulfonic acid was used as received as a monohydrate.

The use of non-nucleophilic solvents such as toluene, p-xylene is optional.

The reactions were carried out under agitation.

### Comparative examples 1a - 1d: Incomplete reacting systems

Control experiments, which did not include all the technical elements of the process of the invention, were conducted.

The progress of the reaction was continuously monitored by ¹H-NMR.

The following Comparative examples were carried out:
- 1a: Equimolar quantities of ethyl cyanoacetate and acetic anhydride or triflic anhydride and 1 ,3,5-trioxane were heated together for 2 hours at 120°C. No trace of monomer was produced.
- 1b: Equimolar quantities of ethyl cyanoacetate and methylene diacetate were heated together for 2 hours at 120°C. No trace of monomer was produced.
- 1c: Equimolar quantities of ethyl cyanoacetate and methylene diacetate were heated together for 1 hour at 120°C in the presence of 5 mol% p-TsOH.H₂O. No trace of monomer was produced and starting materials remained. This Bronsted acid is effective in pre-formation of acylals in independent reactions, but does not catalyse further reaction between active methylene compound and the acylal when the latter is already available.
- 1d: Equimolar quantities of ethyl cyanoacetate and methylene diacetate were heated together for 1 hour at 120°C in the presence of 5 mol% hydroquinone. No trace of monomer was produced and starting materials remained. This substituted phenol compound, hydroquinone, alone does not facilitate reaction with pre-formed methylene diacetate even if it is known to assist in Knoevenagel reactions.

These comparative experiments show that the absence of technical elements of the process of the invention didn't lead to the preparation of electron-deficient olefin monomers.

### Comparative examples 2a - 2d: Incomplete reacting systems

According to US 10,927,071 an electron deficient olefin may be prepared by reacting a cyanoacetate with a source of aldehyde in the presence of certain Lanthanide or transition metal Lewis acid catalysts.

Said patent states that the aldehyde compound is selected from the group paraformaldehyde, formalin, 1,3,5-trioxane, methylene diacetate, dimethoxymethane and acrolein. Some of these reactants, were tested in comparative experiments.
- 2a: InCl₃ used at 1 mol% was examined as a catalyst for a reaction between equimolar quantities of ethyl cyanoacetate and dimethoxymethane. Dimethoxymethane has a boiling point of about 41°C at ambient pressure. So as to enable higher reaction temperatures as well as to attempt to promote a Knoevenagel reaction, acetic anhydride, with a boiling point of about 140°C, was admixed with dimethoxymethane as a reagent and solvent. No reaction occurred and no monomer formation was observed after heating for 2 hours at 120°C. Ethyl cyanoacetate was recovered unconverted. Acetic anhydride did not promote the reaction when dimethoxymethane, as a source of aldehyde, was used as reactant.
- 2b: Another experiment was conducted to test the performance of other examples of sources of (form)aldehyde from those listed above. In an experiment using cyanoacetic acid in place of a cyanoacetate as substrate and paraformaldehyde in place of dimethoxymethane using 5 mol % InCl₃ conducted at 60°C for 2 hours, only viscous solutions resulted that gave various broad, poorly resolved peaks across the 3.3-7.6ppm region in ¹H-NMR spectra indicative of undefined polymer(s) and water/OH. Acetic anhydride was not used in this experiment.
- 2c: Another experiment was conducted using ethyl cyanoacetate as a substrate and 1,3,5-trioxane in equimolar quantities using a catalyst system comprising Lanthanide Lewis acid Yb(OTf)₃, *p*-TsOH and hydroquinone all at 5mol% conducted at 120°C for 2 hours. No monomer was produced even though starting material was 100% converted. Acetic anhydride was not present in this experiment.
- 2d: Similar results to those in 2c were obtained when the post-transition metal catalyst InCl₃ was employed along with *p*-TsOH and hydroquinone as before even by increasing the Lewis acid catalyst concentration to 10mol%, lowering reaction temperature to 60°C and extending reaction time to 18 hours. When acetic anhydride was absent no acylal could form and the used catalyst systems did not facilitate direct production of stable monomer.

### Comparative examples 3a - 3e: Incomplete reacting systems

According to the Examples disclosed in US 9,670,145 (US'145 patent), the active methylene compound *n*-butyl cyanoacetate (BCAc) may be reacted with the acylal methylene diacetate (MDAc) or a source thereof using an ammonium or iminium salt catalyst (*N*-based organocatalyst) with a reaction time of 2 hours and a temperature in the range 120-130°C. The reaction efficiency is independent of the way in which the *N*-based organocatalyst is prepared, but dependent on the way the MDAc is prepared. That patent describes *in situ* preparation of MDAc from paraformaldehyde and acetic anhydride with excess *p*-TsOH.H₂O and in the presence of cyanoacetate which is a three-component approach therein described as the 'direct method' in Example 3 of said patent. MDAc was also pre-formed as a product from an independent reaction using sulfuric acid as catalyst (Example 1 of said patent) and reacted with *n*-butyl cyanoacetate in a two-component approach. When MDAc was prepared according to the former case and used in the *N*-based organocatalysed reaction, the usual selectivity for product monomer *n*-butyl cyanoacrylate (BCA) was disclosed as being high at approximately 70-75%. When the MDAc was pre-formed, the usual selectivity for product monomer *n*-butyl cyanoacrylate (BCA) from the *N*-based organocatalysed reaction was disclosed as very high at about >90%. The two-component reaction delivered the highest yields.

Experiments were performed to establish a reference point for subsequent comparisons firstly by using pure pre-formed MDAc prepared in an independent reaction. MDAc was prepared in pure form by the method of Tomiška *et al., op. cit.,* except using *p*-TsOH.H₂O as catalyst. The pre-formed MDAc was reacted with a variety of different cyanoacetates in an equimolar stoichiometry using the *N*-based organocatalyst prepared as described in the US'145 patent (5 mol% piperazine, 15 mol% *p*-TsOH.H₂O) for a 2-hour reaction time at 120°C. No hydroquinone was used in the reaction. The results for the conversion of starting materials directly to the corresponding cyanoacrylate products, i.e., selectivity specifically to monomer, measured by ¹H-NMR using an internal standard, for this two-component reaction under these conditions are shown in Table I:

**TABLE I**

| **Comparative Example** | **Cyanoacetate Starting material** | **Conversion to cyanoacrylate monomer (% by ¹H-NMR *)** |
|---|---|---|
| 3a | Ethyl- | 69 |
| 3b | *iso*-Propyl- | 85 |
| 3c | *n*-Butyl- | 73 |
| 3d | *n*-Octyl- | 75 |

| | | |
|---|---|---|
| *10 mmol tetrachloroethane used as internal standard | | |

The N-based organocatalysed reaction described in Comparative Examples 3a - 3d above (5mol% piperazine, 15mol% *p*-TsOH.H₂O) was again repeated this time in a three-component reaction by forming MDAc *in situ* and only with ethyl cyanoacetate. No hydroquinone was used in the reaction.

In this three-component experiment (Example 3e) the MDAc was formed by reacting 1,3,5-trioxane recrystalised from a 1:1 mixture of ethyl acetate-petroleum ether, with acetic anhydride and the excess *p*-TsOH.H₂O. Ethyl cyanoacetate, 1,3,5-trioxane and acetic anhydride were employed using 1:1:2 molar equivalents respectively in a mixture. The mixture was heated to 120°C and maintained at this temperature for between 1.5 and 2 hours. The result for the conversion of ethyl cyanoacetate directly to the corresponding ethyl cyanoacrylate, i.e., selectivity to monomer only, measured by ¹H-NMR was only 15%.

### Examples 1a to 1e: Preparation of different cyanoacrylates

In the following examples equimolar quantities of specific cyanoacetates and 1,3,5-trioxane were reacted with two molar equivalents of acetic anhydride at 120°C for 1.5-2 hours in the presence of Lewis acid indium trichloride (InCl₃) catalyst at a 1mol % concentration relative to the quantity of cyanoacetate and 5 mol% hydroquinone and 5 mol% of *p*-TsOH.H₂O. Examples 1 to 4 were solventless, whereas in Example 5, the reaction mixture further comprised the solvent *p*-xylene in approximately equal volume measure to the total volume of all the reagents

The reaction mixture was vigorously stirred. The progress of the reaction was continuously monitored by ¹H-NMR using tetrachloroethane as an internal standard. Analysis for the conversion of starting materials directly to the corresponding cyanoacrylate products in the reaction medium, i.e., selectivity specifically to monomer, are shown in Table II and demonstrate how low loadings of a specific Lewis acid catalyst in a catalyst system readily produce a variety of cyanoacrylate monomers directly in high yields in a stable environment but without recourse to pre-formation of MDAc which is a separate reaction requiring additional time:

**TABLE II**

| **Example** | **Cyanoacetate** | **Conversion to cyanoacrylate monomer (% by ¹H-NMR *)** |
|---|---|---|
| 1a | Ethyl- | 63 |
| 1b | *iso*-Propyl- | 69 |
| 1c | *n*-Butyl- | 67 |
| 1d | *n*-Octyl- | 70 |
| 1e | Ethyl- + *p*-xylene | 75 |

| | | |
|---|---|---|
| *10 mmol tetrachloroethane used as internal standard | | |

It can be observed that the selectivity obtained in Example 1 is much superior to the selectivity obtained from an *N*-based organocatalysed equivalent three-component reaction (Comparative Example 3e).

When 2-methoxyethyl cyanoacetate was used as the active methylene compound the catalyst system was adjusted and comprised InCl₃, *p*-TsOH.H₂O and hydroquinone all at 5 mol% and reaction time was reduced to 1 hour at 120°C. 2-Methoxy cyanoacrylate was produced in a yield of 40% with an 88% conversion of the cyanoacetate without added solvent.

The one-pot three-component reaction was simple and convenient to perform from readily available starting materials and acetic acid by-product was clearly present in the reaction mixture as evidenced by ¹H-NMR.

As shown in Example 1e, the selectivity and conversion were boosted even further to 75% and 90% respectively as measured by ¹H-NMR, when *p*-xylene was added to the reaction mixture as solvent.

The use of other solvents in the InCl₃ catalysed reaction such as 1,4-dioxane was also possible in the two-component approach using pre-formed MDAc and enabled efficient reaction at even lower loadings of InCl₃, such as 0.5 mol%, albeit with longer reaction times of up to 5 hours.

### Examples 2a to 2e: Preparation of ethyl cyanoacrylate using different co-catalysts

Co-catalysts for the three-component embodiment was examined using a catalyst system employing 1 mol% InCl₃, 5 mol% *p*-TsOH.H₂O and various co-catalysts at concentrations indicated in Table III that also includes the result of Examples 1a and 1e for convenience of comparison. The molar ratio of ethyl cyanoacetate to 1,3,5-trioxane to acetic anhydride was 1:1:2 and reaction time was 2 hours at 120°C.

**TABLE III**

| **Example** | **Co-catalyst (mol%)** | **Conversion to cyanoacrylate monomer (% by ¹H-NMR *)** |
|---|---|---|
| Control | None (average of duplicated experiments) | 15 |
| 2a | Phenol (5 mol%) | 48 |
| 2b | *p*-Trifluoromethyl phenol (5mol%) | 35 |
| 2c | *p*-Methoxyphenol (5 mol%) | 33 |
| 2d | Hydroquinone (0.02 mol%) (average of duplicated experiments) | 52.5 |
| 1a | Hydroquinone (5 mol%) | 63 |
| 1e | Hydroquinone (5 mol%) reaction diluted with equal volume of *p*-xylene | 75 |

| | | |
|---|---|---|
| *10 mmol tetrachloroethane used as internal standard | | |

### Examples 3a to 3e: Preparation of ethyl cyanoacrylate using different Lewis acids and loads

Different Lewis acid for the three-component embodiment were examined using a catalyst system also comprising 5 mol% *p*-TsOH.H₂O and 5 mol% of hydroquinone as indicated in Table IV that also includes the results of Examples 1a and 1e for convenience of comparison. The molar ratio of ethyl cyanoacetate to 1,3,5-trioxane to acetic anhydride was 1:1:2 and reaction time was 2 hours at 120°C. In Examples 3d and 3e, 1,3,5-trioxane was recrystallised from a 1:1 ethyl acetate-petroleum ether mixture and the reaction time was reduced to 1.5 hours.

**TABLE IV**

| **Example** | **Lewis acid** | **Conversion to cyanoacrylate monomer (% by ¹H-NMR *)** |
|---|---|---|
| 3a | Yb(OTf)₃ 5 mol% | 14 |
| 3b | Mg(ClO₄)₂ 1 mol% | 34 |
| 3c | GaCl₃ 1 mol% | 50 |
| 3d | InCl₃ 0.5 mol% | 50 |
| 3e | InCl₃ 0.1 mol% | 50 |
| 1a | InCl₃ 1 mol% | 63 |
| 1e | InCl₃ 1mol% + *p*-xylene added | 75 |

| | | |
|---|---|---|
| *10 mmol tetrachloroethane used as internal standard | | |

The results indicate that various Lewis acids can produce monomer in a stable environment using the three-component embodiment.

It can be further observed that the results indicate efficiency of the catalyst system for the reaction even using loadings as low as 0.1 mol% for the InCl₃ component.

### Example 4: Preparation of ethyl cyanoacrylate

A mixture comprising 10 g ethyl cyanoacetate, 9 g 1,3,5-trioxane, and 20 g of acetic anhydride was stirred and heated to 120°C for 5 hours in the presence of the catalyst system comprising 1 mol% InCl₃, 5 mol% hydroquinone and 5 mol% *p*-TsOH.H₂O.

At the end of the reaction a fractional distillation was performed directly on the reaction mixture once ethyl cyanoacetate was completely consumed as followed by ¹H-NMR. There was no need to separate of catalyst before distillation. Acetic acid by-product distilled first. Pure ethyl cyanoacrylate, free from ethyl cyanoacetate as shown by ¹H-NMR in acetone-d₆, was isolated in the bulk state after distilling at 110°C and 10 mbar, without using volatile acidic stabilisers. The yield of pure ethyl cyanoacrylate was higher than 60%.

### Example 5: Preparation of ethyl cyanoacrylate

A mixture comprising 1100 mg ethyl cyanoacetate, 1300 mg methylene diacetate was stirred and heated to 120°C for 1 hours in the presence of the catalyst system comprising 5 mol% hydroquinone and 5 mol% *p*-TsOH.H₂O.

Ethyl cyanoacrylate was obtained with a conversion of 46%, according to ¹H-NMR data.

## Claims

1. A process for preparing electron-deficient olefin monomers, **characterized in that** an active methylene compound of general formula (III),
EWG-CH₂-Z¹ (III)
wherein the two electron withdrawing groups represented by EWG and Z¹, are selected from nitrile, carboxylic ester, and carboxylic acid functional groups,
is reacted with either
a) a mixture of a source of formaldehyde and a carboxylic acid anhydride of general formula (IV), 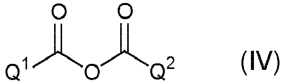 wherein Q¹ and Q² are independently selected from linear and branched C₁-C₄ alkyl, optionally with H atoms substituted by F atoms, preferably are Me or Et, and more preferably are Me, in the presence of a catalyst system that comprises:
i) at least one acid, or,
ii) at least one acid and at least one co-catalyst,
wherein the acid is selected from the group of Lewis and Bronsted acids, and the at least one co-catalyst is selected from the group of phenolic compounds, with one or more substituents, and quinone compounds, or
b) a methylene diester of general formula (V), 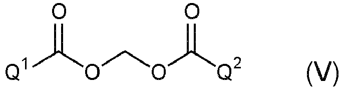 wherein Q1 and Q2 are independently selected from C₁-C₄ linear and branched alkyl groups,
in the presence of a catalyst system that comprises a Brønsted acid and at least one co-catalyst selected from the group of phenolic compounds, with one or more substituents, and quinone compounds.

2. The process according to claim 1, **characterized in that** the active methylene compound of general formula (III),
EWG-CH₂-Z¹ (III)
wherein the two electron withdrawing groups represented by EWG and Z¹, are selected from nitrile, carboxylic ester, and carboxylic acid functional groups,
is reacted with a mixture of a source of formaldehyde and a carboxylic acid anhydride of general formula (IV), 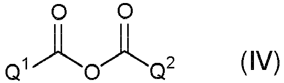 wherein Q¹ and Q² are independently selected from linear and branched C₁-C₄ alkyl, optionally with H atoms substituted by F atoms, preferably are Me or Et, and more preferably are Me,
in the presence of a catalyst system that comprises:
i) at least one acid, or,
ii) at least one acid and at least one co-catalyst,
wherein the acid is selected from the group of Lewis and Brønsted acids, and the at least one co-catalyst is selected from the group of phenolic compounds, with one or more substituents, and quinone compounds.

3. The process according to claims 1 or 2, **characterized in that** the active methylene compound of formula (III) comprises a nitrile and a carboxylic ester as electron withdrawing functional groups.

4. The process according to any one of claims 1 to 3, **characterized in that** the source of formaldehyde is selected from dry formaldehyde gas, paraformaldehyde, and 1,3,5-trioxane, preferably it is 1,3,5-trioxane.

5. The process according to any one of claims 1 to 4, **characterized in that** the catalyst system is selected from a Lewis acid, preferably a post-transition metal Lewis acid; a combination of a Lewis acid, preferably a post-transition metal Lewis acid, and a Brønsted acid; a combination of a Lewis acid, preferably a post-transition metal Lewis acid, and a co-catalyst selected from phenolic compounds, with one or more substituents, quinone compounds, and mixtures thereof; a combination of a Brønsted acid and a co-catalyst selected from phenolic compounds, with one or more substituents, quinone compounds, and mixtures thereof, and a combination of a Lewis acid, preferably a post-transition metal Lewis acid, and a Bronsted acid and a co-catalyst selected from phenolic compounds, with one or more substituents, quinone compounds, and mixtures thereof, preferably it is a combination of Lewis acid, preferably a post-transition metal Lewis acid, a Brønsted acid and a co-catalyst selected from phenolic compounds, with one or more substituents, quinone compounds, and mixtures thereof.

6. The process according to any one of claims 1 to 5, **characterized in that** the co-catalyst is selected from phenol, hydroquinone, *p*-methoxyphenol, *p*-trifluoromethyl phenol, 1,4-benzoquinone, 1,4-naphthoquinone, and mixtures thereof, preferably, it is hydroquinone.

7. The process according to any one of claims 1 to 6, **characterized in that** the Lewis acid is selected from transition metals halides, post-transition metal-based halides, preferably trihalides, alkali earth metal-based perchlorates, and Lanthanide metal based, preferably selected from the group of post-transition metals, more preferably from indium (III) trihalide and gallium (III) trihalide, preferably trichlorides, and yet more preferably the Lewis acid is InCl₃.

8. The process according to any one of claims 1 to 7, **characterized in that** the Brønsted acid is selected from *p*-toluene sulfonic acid, benzene sulfonic acid, and aralkylsulfonic acids, preferably it is *p*-toluene sulfonic acid, whether anhydrous form or as monohydrate.

9. The process according to any one of claims 1 to 8, **characterized in that** it comprises the use of stabilisers selected from acid stabilisers, preferably selected from boron trifluoride etherate, SO₂, trifluoracetic acid, more preferably boron trifluoride etherate; and radical stabilisers, preferably selected from 2,2'-methylenebis-(6-*tert*-buthyl-*p*-cresol), pentaerythritol tetrakis[3-[3,5-di-*tert*-butyl-4-hydroxyphenyl]propionate hydroquinone, 4-methoxyphenol, butylated hydroxyanisole, and butylated hydroxytoluene.

10. The process according to claim 1, **characterized in that** the active methylene compound of general formula (III),
EWG-CH₂-Z¹ (III)
wherein the two electron withdrawing groups represented by EWG and Z¹, are selected from nitrile, carboxylic ester, and carboxylic acid functional groups,
is reacted with a methylene diester of general formula (V), 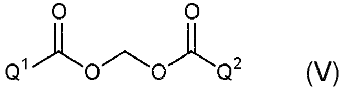 wherein Q1 and Q2 are independently selected from C₁-C₄ linear and branched alkyl groups,
in the presence of a catalyst system that comprises a Brønsted acid and at least one co-catalyst selected from the group of phenolic compounds, with one or more substituents, and quinone compounds.

11. The process according to claim 10, **characterized in that** it is used an active methylene compound of general formula (III) as defined in any one of claims 3 to 5.

12. The process according to claims 10 or 11, **characterized in that** the methylene diester is methylene diacetate.

13. The process according to any one of claims 10 to 12, **characterized in that** it is used a Brønsted acid as defined in claim 8.

14. The process according to any one of claims 10 to 13, **characterized in that** it is used a co-catalyst as defined in claim 6.

15. The process according to any one of claims 10 to 14, **characterized in that** it comprises stabilisers as defined in claim 9.

## Patentansprüche

1. Verfahren zur Herstellung von elektronenarmen Olefinmonomeren, **dadurch gekennzeichnet, dass** eine aktive Methylenverbindung der allgemeinen Formel (III),
EWG-CH₂-Z¹ (III)
in der die zwei durch EWG und Z¹ dargestellten elektronenziehenden Gruppen aus Nitril-, Carbonsäureester- und Carbonsäure-Funktionsgruppen ausgewählt sind,
entweder mit
a) einer Mischung aus einer Formaldehydquelle und einem Carbonsäureanhydrid der allgemeinen Formel (IV), 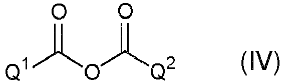 in der Q¹ und Q² unabhängig voneinander aus linearem und verzweigtem C₁-C₄-Alkyl, gegebenenfalls mit durch F-Atome substituierten H-Atomen, ausgewählt sind, bevorzugt Me oder Et sind und besonders bevorzugt Me sind,
in Gegenwart eines Katalysatorsystems, das Folgendes umfasst:
i) mindestens eine Säure, oder
ii) mindestens eine Säure und mindestens einen Cokatalysator,
wobei die Säure aus der Gruppe der Lewis- und Brønsted-Säuren ausgewählt ist und der mindestens eine Cokatalysator aus der Gruppe der Phenolverbindungen mit einem oder mehreren Substituenten und Chinonverbindungen ausgewählt ist, oder
b) einem Methylendiester der allgemeinen Formel (V), 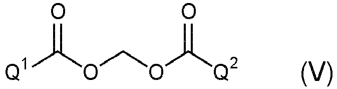 in der Q1 und Q2 unabhängig voneinander aus linearen und verzweigten C₁-C₄-Alkylgruppen ausgewählt sind,
in Gegenwart eines Katalysatorsystems, das eine Brønsted-Säure und mindestens einen Cokatalysator umfasst, ausgewählt aus der Gruppe der Phenolverbindungen mit einem oder mehreren Substituenten und Chinonverbindungen, umgesetzt wird.

2. Verfahren nach Anspruch 1, **dadurch gekennzeichnet, dass** die aktive Methylenverbindung der allgemeinen Formel (III),
EWG-CH₂-Z¹ (III)
in der die zwei durch EWG und Z¹ dargestellten elektronenziehenden Gruppen aus Nitril-, Carbonsäureester- und Carbonsäure-Funktionsgruppen ausgewählt sind,
mit einer Mischung aus einer Formaldehydquelle und einem Carbonsäureanhydrid der allgemeinen Formel (IV), 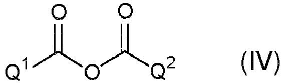 in der Q¹ und Q² unabhängig voneinander aus linearem und verzweigtem C₁-C₄-Alkyl, gegebenenfalls mit durch F-Atome substituierten H-Atomen, ausgewählt sind, bevorzugt Me oder Et sind und besonders bevorzugt Me sind,
in Gegenwart eines Katalysatorsystems, das Folgendes umfasst:
i) mindestens eine Säure, oder
ii) mindestens eine Säure und mindestens einen Cokatalysator,
wobei die Säure aus der Gruppe der Lewis- und Brønsted-Säuren ausgewählt ist und der mindestens eine Cokatalysator aus der Gruppe der Phenolverbindungen mit einem oder mehreren Substituenten und Chinonverbindungen ausgewählt ist, umgesetzt wird.

3. Verfahren nach Anspruch 1 oder 2, **dadurch gekennzeichnet, dass** die aktive Methylenverbindung der Formel (III) ein Nitril und einen Carbonsäureester als elektronenziehende Funktionsgruppen umfasst.

4. Verfahren nach einem der Ansprüche 1 bis 3, **dadurch gekennzeichnet, dass** die Formaldehydquelle aus trockenem Formaldehydgas, Paraformaldehyd und 1 ,3,5-Trioxan, bevorzugt 1 ,3,5-Trioxan, ausgewählt ist.

5. Verfahren nach einem der Ansprüche 1 bis 4, **dadurch gekennzeichnet, dass** das Katalysatorsystem aus einer Lewis-Säure, bevorzugt einer Post-Übergangsmetall-Lewis-Säure, einer Kombination aus einer Lewis-Säure, bevorzugt einer Post-Übergangsmetall-Lewis-Säure, und einer Brønsted-Säure; einer Kombination aus einer Lewis-Säure, bevorzugt einer Post-Übergangsmetall-Lewis-Säure, und einem Cokatalysator, ausgewählt aus Phenolverbindungen mit einem oder mehreren Substituenten, Chinonverbindungen und Mischungen davon; einer Kombination aus einer Brønsted-Säure und einem Cokatalysator, ausgewählt aus Phenolverbindungen mit einem oder mehreren Substituenten, Chinonverbindungen und Mischungen davon, und einer Kombination aus einer Lewis-Säure, bevorzugt einer Post-Übergangsmetall-Lewis-Säure, und einer Brønsted-Säure und einen Cokatalysator, ausgewählt aus Phenolverbindungen mit einem oder mehreren Substituenten, Chinonverbindungen und Mischungen davon, ausgewählt ist, wobei eine Kombination aus Lewis-Säure, bevorzugt einer Post-Übergangsmetall-Lewis-Säure, einer Brønsted-Säure und einem ausgewählten Cokatalysator aus phenolischen Verbindungen mit einem oder mehreren Substituenten, Chinonverbindungen und Mischungen davon, bevorzugt wird.

6. Verfahren nach einem der Ansprüche 1 bis 5, **dadurch gekennzeichnet, dass** der Cokatalysator aus Phenol, Hydrochinon, *p*-Methoxyphenol, *p-*Trifluormethylphenol, 1,4-Benzochinon, 1,4-Naphthochinon und Mischungen davon ausgewählt ist, wobei Hydrochinon bevorzugt wird.

7. Verfahren nach einem der Ansprüche 1 bis 6, **dadurch gekennzeichnet, dass** die Lewis-Säure aus Übergangsmetallhalogeniden, Halogeniden auf Post-Übergangsmetallbasis, bevorzugt Trihalogeniden, Perchloraten auf Erdalkalimetallbasis und Lanthanidmetallbasis ausgewählt ist, bevorzugt ausgewählt aus der Gruppe der Post-Übergangsmetalle, besonders bevorzugt aus Indium(III)-Trihalogenid und Gallium(III)-Trihalogenid, bevorzugt Trichloride, und noch bevorzugter die Lewis-Säure InCl₃ ist.

8. Verfahren nach einem der Ansprüche 1 bis 7, **dadurch gekennzeichnet, dass** die Brønsted-Säure aus *p*-Toluolsulfonsäure, Benzolsulfonsäure und Aralkylsulfonsäuren ausgewählt ist, wobei *p*-Toluolsulfonsäure, entweder in wasserfreier Form oder als Monohydrat, bevorzugt wird.

9. Verfahren nach einem der Ansprüche 1 bis 8, **dadurch gekennzeichnet, dass** es die Verwendung von Stabilisatoren, ausgewählt aus Säurestabilisatoren, bevorzugt ausgewählt aus Bortrifluoridetherat, SO₂, Trifluoressigsäure, besonders bevorzugt Bortrifluoridetherat; und Radikalstabilisatoren, bevorzugt ausgewählt aus 2,2'-Methylenbis-(6-*tert*-Butyl-*p*-Kresol), Pentaerythritol-Tetrakis[3-[3,5-di-*tert*-Butyl-4-Hydroxyphenyl]propionathydrochinon, 4-Methoxyphenol, butyliertes Hydroxyanisol und butyliertes Hydroxytoluol, umfasst.

10. Verfahren nach Anspruch 1, **dadurch gekennzeichnet, dass** die aktive Methylenverbindung der allgemeinen Formel (III),
EWG-CH₂-Z¹ (III)
in der die zwei durch EWG und Z¹ dargestellten elektronenziehenden Gruppen aus Nitril-, Carbonsäureester- und Carbonsäure-Funktionsgruppen ausgewählt sind,
mit einem Methylendiester der allgemeinen Formel (V), 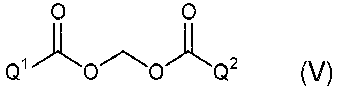 in der Q1 und Q2 unabhängig voneinander aus linearen und verzweigten C₁-C₄-Alkylgruppen ausgewählt sind,
in Gegenwart eines Katalysatorsystems, das eine Brønsted-Säure und mindestens einen Cokatalysator umfasst, ausgewählt aus der Gruppe der Phenolverbindungen mit einem oder mehreren Substituenten und Chinonverbindungen, umgesetzt wird.

11. Verfahren nach Anspruch 10, **dadurch gekennzeichnet, dass** eine aktive Methylenverbindung der allgemeinen Formel (III) wie in einem der Ansprüche 3 bis 5 definiert verwendet wird.

12. Verfahren nach Anspruch 10 oder 11, **dadurch gekennzeichnet, dass** der Methylendiester Methylendiacetat ist.

13. Verfahren nach einem der Ansprüche 10 bis 12, **dadurch gekennzeichnet, dass** eine Brønsted-Säure wie in Anspruch 8 definiert verwendet wird.

14. Verfahren nach einem der Ansprüche 10 bis 13, **dadurch gekennzeichnet, dass** ein Cokatalysator wie in Anspruch 6 definiert verwendet wird.

15. Verfahren nach einem der Ansprüche 10 bis 14, **dadurch gekennzeichnet, dass** es Stabilisatoren wie in Anspruch 9 definiert umfasst.

## Revendications

1. Procédé de préparation de monomères oléfiniques déficients en électrons, **caractérisé en ce qu'**un composé méthylène actif de formule générale (III),
EWG-CH₂-Z¹ (III)
dans laquelle les deux groupes attracteurs d'électrons représentés par EWG et Z¹, sont sélectionnés parmi les groupes fonctionnels nitrile, ester carboxylique et acide carboxylique,
est réagi avec soit
a) un mélange d'une source de formaldéhyde et d'un anhydride d'acide carboxylique de formule générale (IV), 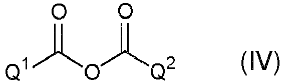 dans laquelle Q¹ et Q² sont indépendamment sélectionnés parmi les alkyles en C₁-C₄ linéaires et ramifiés, éventuellement avec des atomes H substitués par des atomes F, sont de préférence Me ou Et, et plus préférablement sont Me,
en présence d'un système catalytique qui comprend :
i) au moins un acide, ou,
ii) au moins un acide et au moins un co-catalyseur,
dans lequel l'acide est sélectionné dans le groupe des acides de Lewis et de Brønsted, et l'au moins un co-catalyseur est sélectionné dans le groupe des composés phénoliques, avec un ou plusieurs substituants, et des composés quinones, ou
b) un diester de méthylène de formule générale (V), 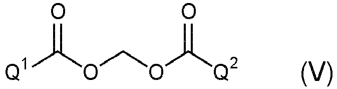 dans laquelle Q1 et Q2 sont indépendamment sélectionnés parmi les groupes alkyle en C₁-C₄ linéaires et ramifiés,
en présence d'un système catalytique qui comprend un acide de Brønsted et au moins un co-catalyseur sélectionné dans le groupe des composés phénoliques, avec un ou plusieurs substituants, et des composés quinones.

2. Procédé selon la revendication 1, **caractérisé en ce que** le composé méthylène actif de formule générale (III),
EWG-CH₂-Z¹ (III)
dans laquelle les deux groupes attracteurs d'électrons représentés par EWG et Z¹, sont sélectionnés parmi les groupes fonctionnels nitrile, ester carboxylique et acide carboxylique,
est réagi avec un mélange d'une source de formaldéhyde et d'un anhydride d'acide carboxylique de formule générale (IV), 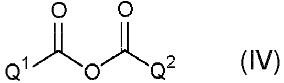 dans laquelle Q¹ et Q² sont indépendamment sélectionnés parmi les alkyles en C₁-C₄ linéaires et ramifiés, éventuellement avec des atomes H substitués par des atomes F, sont de préférence Me ou Et, et plus préférablement sont Me,
en présence d'un système catalytique qui comprend :
i) au moins un acide, ou,
ii) au moins un acide et au moins un co-catalyseur,
dans lequel l'acide est sélectionné dans le groupe des acides de Lewis et de Brønsted, et l'au moins un co-catalyseur est sélectionné dans le groupe des composés phénoliques, avec un ou plusieurs substituants, et des composés quinones.

3. Procédé selon la revendication 1 ou 2, **caractérisé en ce que** le composé méthylène actif de formule (III) comprend un nitrile et un ester carboxylique comme groupes fonctionnels attracteurs d'électrons.

4. Procédé selon l'une quelconque des revendications 1 à 3, **caractérisé en ce que** la source de formaldéhyde est sélectionnée parmi le formaldéhyde gazeux sec, le paraformaldéhyde et le 1,3,5-trioxane, est de préférence le 1,3,5-trioxane.

5. Procédé selon l'une quelconque des revendications 1 à 4, **caractérisé en ce que** le système catalytique est sélectionné parmi un acide de Lewis, de préférence un acide de Lewis de métal de post-transition ; une combinaison d'un acide de Lewis, de préférence d'un acide de Lewis de métal de post-transition et d'un acide de Brønsted ; une combinaison d'un acide de Lewis, de préférence d'un acide de Lewis de métal de post-transition, et d'un co-catalyseur sélectionné parmi les composés phénoliques, avec un ou plusieurs substituants, les composés quinones et leurs mélanges ; une combinaison d'un acide de Brønsted et d'un co-catalyseur sélectionné parmi les composés phénoliques, avec un ou plusieurs substituants, les composés quinones, et leurs mélanges, et une combinaison d'un acide de Lewis, de préférence d'un acide de Lewis de métal de post-transition, et d'un acide de Brønsted et d'un co-catalyseur choisi parmi les composés phénoliques, avec un ou plusieurs substituants, les composés quinones et leurs mélanges, de préférence est une combinaison d'acide de Lewis, de préférence d'un acide de Lewis de métal de post-transition, d'un acide de Brønsted et d'un co-catalyseur sélectionné parmi les composés phénoliques, avec un ou plusieurs substituants, les composés quinones et leurs mélanges.

6. Procédé selon l'une quelconque des revendications 1 à 5, **caractérisé en ce que** le co-catalyseur est sélectionné parmi le phénol, l'hydroquinone, le *p-*méthoxyphénol, le *p*-trifluorométhylphénol, la 1,4-benzoquinone, la 1,4-naphtoquinone et leurs mélanges, de préférence, est l'hydroquinone.

7. Procédé selon l'une quelconque des revendications 1 à 6, **caractérisé en ce que** l'acide de Lewis est sélectionné parmi les halogénures de métaux de transition, les halogénures à base de métaux post-transition, de préférence les trihalogénures, les perchlorates à base de métaux alcalino-terreux et les lanthanides à base de métaux, de préférence sélectionné dans le groupe des métaux de post-transition, plus préférablement parmi le trihalogénure d'indium (III) et le trihalogénure de gallium (III), de préférence les trichlorures, et encore plus préférablement l'acide de Lewis est InCl₃.

8. Procédé selon l'une quelconque des revendications 1 à 7, **caractérisé en ce que** l'acide de Brønsted est sélectionné parmi l'acide *p*-toluène sulfonique, l'acide benzène sulfonique et les acides aralkylsulfoniques, de préférence est l'acide *p*-toluène sulfonique, que ce soit sous forme anhydre ou sous forme monohydratée.

9. Procédé selon l'une quelconque des revendications 1 à 8, **caractérisé en ce qu'**il comprend l'utilisation de stabilisateurs sélectionnés parmi les stabilisateurs acides, de préférence sélectionnés parmi l'éthérate de trifluorure de bore, SO₂, l'acide trifluoracétique, plus préférablement l'éthérate de trifluorure de bore ; et les stabilisateurs radicalaires, de préférence sélectionnés parmi le 2,2'-méthylènebis-(6-*tert*-butyle-*p*-crésol), le pentaérythritol tétrakis[3-[3,5-di-*tert-*butyl-4-hydroxyphényl]propionate d'hydroquinone, le 4-méthoxyphénol, l'hydroxyanisole butylé et l'hydroxytoluène butylé.

10. Procédé selon la revendication 1, **caractérisé en ce que** le composé méthylène actif de formule générale (III),
EWG-CH₂-Z¹ (III)
dans laquelle les deux groupes attracteurs d'électrons représentés par EWG et Z¹, sont sélectionnés parmi les groupes fonctionnels nitrile, ester carboxylique et acide carboxylique,
est réagi avec un diester de méthylène de formule générale (V), 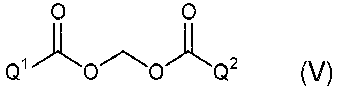 dans laquelle Q1 et Q2 sont indépendamment sélectionnés parmi les groupes alkyle en C₁-C₄ linéaires et ramifiés,
en présence d'un système catalytique qui comprend un acide de Brønsted et au moins un cocatalyseur sélectionné dans le groupe des composés phénoliques, avec un ou plusieurs substituants, et des composés quinones.

11. Procédé selon la revendication 10, **caractérisé en ce qu'**il est utilisé un composé méthylène actif de formule générale (III) telle que définie dans l'une quelconque des revendications 3 à 5.

12. Procédé selon la revendication 10 ou 11, **caractérisé en ce que** le diester de méthylène est le diacétate de méthylène.

13. Procédé selon l'une quelconque des revendications 10 à 12, **caractérisé en ce qu'**on utilise un acide de Brønsted tel que défini dans la revendication 8.

14. Procédé selon l'une quelconque des revendications 10 à 13, **caractérisé en ce qu'**on utilise un co-catalyseur tel que défini dans la revendication 6.

15. Procédé selon l'une quelconque des revendications 10 à 14, **caractérisé en ce qu'**il comprend des stabilisateurs tels que définis dans la revendication 9.
